# EUROPÄISCHE PATENTSCHRIFT

(11) **EP 2 862 531 B1**
(45) Veröffentlichungstag und Bekanntmachung des Hinweises auf die Patenterteilung: **01.04.2020**
(21) Anmeldenummer: 14189785.0
(22) Anmeldetag: 21.10.2014
(51) Int. Cl.: A61B 18/04, A61B 18/16

(54) **Instrumententestanordnung**
Instrument test arrangement
Dispositif de test d'instruments

(30) Priorität: 21.10.2013 EP 13189566
(43) Veröffentlichungstag der Anmeldung: 22.04.2015
(73) Patentinhaber: Erbe Elektromedizin GmbH, 72072 Tübingen (DE)
(72) Erfinder: Nold, Bernhard Tobias, Dr. rer. nat., 72070 Tübingen (DE); Zenker, Matthias, 72074 Tübingen (DE); Selig, Peter, 72379 Hechingen (DE)
(74) Vertreter: Rüger Abel Patentanwälte PartGmbB

(56) Entgegenhaltungen:
- EP-A1- 1 764 057
- EP-A1- 2 537 479
- WO-A1-2013/139945

## Beschreibung

Die Erfindung betrifft eine Instrumententestanordnung, insbesondere für medizinische Instrumente zur Argon-Plasma-Koagulation.

Im täglichen Betrieb gängige Stellen, gegen die der Anwender die Zündfähigkeit des Koagulationsinstruments überprüft, sind Metallteile von Einrichtungen des Operationssaals. Aufgrund einer solchen unzulässigen Überprüfung an irgendwelchen mehr oder weniger gut geerdeten Metallteilen sind Verletzungen von Patient und Personal sowie Beschädigungen elektrischer und elektronischer Einrichtungen möglich.

Argon-Plasma-Koagulationsinstrumente werden zur Erzeugung eines Funkens von einem HF-Generator gespeist. Vor dem Einsatz des Koagulationsinstruments am Patienten ist es oftmals erwünscht, die Funktionsfähigkeit, insbesondere die Zündfähigkeit des Koagulationsinstruments zu überprüfen, indem ein Testfunken erzeugt wird.

Aus der EP 1 764 057 A1 ist ein Adapterstecker bekannt, der zwischen den Neutralleiteranschluss des Generators und den Neutralstecker der Patientenelektrode in den elektrischen Kreis einzufügen ist. Dieser Zündtestadapter weist einen leitfähigen Bereich auf, an dem das plasmachirurgische Instrument auf Funktion getestet werden kann. Zwischen dem leitfähigen Bereich und dem Neutralleiter des Steckers kann ein Widerstand vorgesehen sein.

Davon ausgehend ist es Aufgabe der Erfindung, ein Konzept anzugeben, mit dem sich auf komfortable Weise die Funktionsfähigkeit, insbesondere die Zündfähigkeit eines Instruments oder einer Sonde vor dem Einsatz am Patienten testen lässt.

Diese Aufgabe wird mit einer Instrumententestanordnung nach Anspruch 1 gelöst, mit der sich die Funktionsfähigkeit eines medizinischen HF-chirurgischen Instruments, insbesondere eines Instruments zur Argon-Plasma-Koagulation gefahrlos testen lässt. Weitere bevorzugte Ausführungsformen sind in den abhängigen Ansprüchen aufgelistet.

Die Instrumententestanordnung kann als modifiziertes Neutralleiterkabel, als Zusatzteil zu einem Neutralleiterkabel, beispielsweise als Aufsatz oder als Zwischenstecker ausgebildet sein. Es kann auch an dem Neutralelektrodenanschluss oder an dem speisenden Gerät selbst ausgebildet sein.

Die Instrumententestanordnung weist eine Elektrodenanordnung mit mindestens einer Zündtestelektrode auf. Das Neutralleiterkabel oder das Gerät weist wenigstens zwei Neutralleiter auf, die gegeneinander isoliert sind und über die an dem Patienten anzubringende Neutral-Teilelektroden angeschlossen sind. Vorzugsweise ist die Zündtestelektrode elektrisch mit wenigstens zwei Neutralleitern eines Neutralleiterkabels oder eines Geräts verbunden.

Die Instrumententestanordnung kann eine einzige Zündtestelektrode oder zwei oder mehrere Zündtestelektroden aufweisen. Eine Zündtestelektrode der Instrumententestanordnung kann aus mehreren untereinander galvanisch verbundenen Abschnitten oder Teilen zusammengesetzt sein. Die Abschnitte oder Teile der Zündtestelektrode können insbesondere fest verdrahtet oder Gleichstrom leitend miteinander verbunden sein. Eine solche Verbindung kann nach der Herstellung der Abschnitte oder Teile der Zündtestelektrode, beispielsweise durch eine Drahtverbindung, geschaffen sein. Eine einteilige Zündtestelektrode kann Materialstege oder - abschnitte aufweisen, die unterschiedliche Abschnitte der Zündtestelektrode miteinander verbinden. Eine Zündtestelektrode mit mehreren Abschnitten oder Teilen, die untereinander verbunden sind, kann sich aus Sicht des Funkens als mehrere Zündtestelektroden darstellen.

Die elektrische Verbindung der Zündtestelektroden mit den Neutralleitern, d.h. die elektrische Kopplung, weist beispielsweise eine kapazitive und/oder resistive Charakteristik auf. Eine Kopplung, insbesondere eine kapazitive Kopplung, kann beispielsweise dadurch geschaffen sein, dass die Zündtestelektrode das Neutralleiterkabel umgibt oder wenigstens abschnittsweise entlang des Umfangs und/oder der Längserstreckung des Neutralleiterkabels mit vorzugsweise festem Abstand zu diesem angeordnet ist. Diskrete kapazitive Bauelemente zwischen dem Neutralleiter und der Zündtestelektrode können so gegebenenfalls entfallen. Die Neutralleiter bilden mit der Zündtestelektrode eine Kondensatoranordnung mit dem Isolationsmaterial des Kabels als Dielektrikum.

Bei der Zündtestelektrode kann es sich beispielsweise um einen metallischen Ring oder eine Manschette handeln, der das Neutralleiterkabel umgibt. Der Ring kann beispielsweise auf dem Neutralleiterkabel aufsitzen oder in die Kabelisolierung eingearbeitet sein. Die Zündtestelektrode kann in diesen Fällen kapazitiv durch die Kabelisolation als Dielektrikum mit den Neutralleitern koppeln. Die Zündtestelektrode kann auch als hervorstehende Kugel ausgebildet sein oder eine solche aufweisen.

Bei der Zündtestelektrode kann es sich um eine Metallfolie handeln. Eine solche kann auf das Neutralleiterkabel aufgebracht, beispielsweise aufgeklebt sein. Die Zündtestelektrode kann durch eine Metallisierung oder eine Metallbeschichtung, beispielsweise eines Neutralleiterkabelabschnitts oder eines Gehäuses einer als Zwischenstecker ausgebildeten Instrumententestanordnung, gebildet sein. Die Metallisierung oder Beschichtung kann z.B. ring-, spiral- oder streifenförmig um bzw. auf das Neutralleiterkabel aufgebracht sein. Beispielsweise kann der Kabelmantel außen wenigstens abschnittsweise metallisiert oder beschichtet sein. Eine spiralförmige Zündtestelektrode kann beispielsweise auch durch einen gebogenen Metallstreifen oder Draht gebildet sein, der sich um das Neutralleiterkabel herumwindend angeordnet ist.

Bevorzugt weist die Instrumententestanordnung eine Testfläche auf, die der Zündtestelektrode zugeordnet ist. Beispielsweise kann es sich bei der Testfläche um eine Fläche der Zündtestelektrode handeln. Der Verwender des chirurgischen Instruments bringt das Instrument in die Nähe der oder an die Testfläche, um die Zündfähigkeit und sonstige Funktionsfähigkeit des Instruments zu überprüfen, die der Verwender an Hand eines von dem Instrument auf die Testfläche überspringenden Funkens feststellen kann. In einer bevorzugten Ausführungsform ist die Testfläche konvex, d.h. nach außen gewölbt, oder die Testfläche weist einen konvexen Abschnitt auf.

Die Testfläche und/oder die Zündtestelektrode ist bevorzugt auf oder an einem konvexen Träger angeordnet. Durch die Anordnung an einem nach außen gewölbten Träger ist die Zündtestelektrode bzw. die Testfläche besonders leicht zugänglich und leicht zu reinigen. Eine Zündtestelektrode mit einer konvexen Testfläche kann beispielsweise durch einen kugel- oder kugelabschnittförmigen Fortsatz oder Aufsatz geschaffen sein.

In einer bevorzugten Ausführungsform weist die Zündtestelektrode eine zusammenhängende Testfläche auf. Durch die unterbrechungsfreie Ausführung der Testfläche wird eine besonders gut zu reinigende Fläche geschaffen.

Die an einem Kabel angebrachte Zündtestelektrode kann flexibel ausgebildet sein, z.B. indem sie als Metallgewebeschlauch ausgebildet ist, der das Neutralleiterkabel wenigstens abschnittsweise umgibt. Die Zündtestelektrode kann auf den Neutralleiter verschieblich oder ortsfest angeordnet sein.

Zwischen den Neutralleitern ist vorzugsweise eine Splitschaltung angeordnet. Diese dient dazu, den zwischen den Teilelektroden der Neutralelektrode herrschenden ohmschen oder komplexen Widerstand zu messen, um die korrekte Befestigung der Teilelektroden an dem Patienten zu verifizieren. Vorzugsweise koppelt die Zündtestelektrode zu den jeweiligen Neutralleitern mit gleicher oder höchstens leicht unterschiedlicher Impedanz. Unter einem nur leichten Unterschied der Impedanzen wird ein Unterschied verstanden, der bei Beaufschlagung der Zündtestelektrode mit HF-Energie nicht zu so unterschiedlichen Strömen in den Neutralleitern führt, dass die Splitschaltung auslöst, also fälschlicherweise mangelnden Kontakt der Teilelektroden der Neutralelektrode zu dem Körper des Patienten anzeigt.

Die Zündtestelektrode kann mit einem eine Impedanz aufweisenden diskreten Bauelement mit dem Neutralleiter verbunden sein. Beispielsweise kann die Zündtestelektrode mit einem ersten eine Impedanz aufweisenden Bauelement mit einem ersten Neutralleiter verbunden und mit einem zweiten eine Impedanz aufweisenden Bauelement mit einem zweiten Neutralleiter verbunden sein. Die Impedanzen der Bauelemente sind vorzugsweise gleich oder höchstens leicht unterschiedlich in dem oben angegebenen Sinne. Bei einem Bauelement kann es sich beispielsweise um einen linearen oder nichtlinearen Widerstand oder um ein kapazitives Bauelement handeln. Es können auch Bauelemente mit einem ohmschen und kapazitiven Anteil an der Impedanz oder Halbleiterbauelemente eingesetzt werden. Um eine Kopplung der Zündtestelektrode zu den jeweiligen Neutralleitern mit gleicher oder höchstens leicht unterschiedlicher Impedanz zu erreichen, weisen die Bauelemente zur Kopplung an den jeweiligen Neutralleiter vorzugsweise gleiche elektrische Kennwerte auf.

In einer Ausführungsform sind zwei Neutralleiter über eine serielle Anordnung von zwei Kapazitäten miteinander verbunden, wobei die Zündtestelektrode elektrisch zwischen den beiden Kapazitäten kontaktiert ist.

Vorzugsweise ist die Gesamtkapazität zwischen den Neutralleitern in der Instrumententestanordnung, beispielsweise in einer als Zwischenstecker oder Gerätestecker des Neutralleiterkabels ausgeführten Form, kleiner oder gleich 1 nF. Vorzugsweise ist die durch Kopplungskapazitäten zwischen der Zündtestelektrode und Neutralleitern erzeugte zusätzliche Kapazität zwischen den Neutralleitern kleiner oder gleich 1 nF. Die Instrumententestanordnung trägt zu der Kapazität zwischen den Neutralleitern vorzugsweise höchstens so viel bei, dass zusätzlich zu der Möglichkeit einen Zündtest vorzunehmen, eine sichere Feststellung der Anlage der Teilelektroden der Neutralelektrode an dem Körper des Patienten durch eine Impedanzmessung zwischen den Teilelektroden ermöglicht wird. Insbesondere ist die Instrumententestanordnung vorzugsweise mit einer entsprechenden Splitschaltung verträglich.

Weitere Einzelheiten vorteilhafter Ausführungsformen der Erfindung sind Gegenstand von Ansprüchen der Beschreibung oder der Zeichnung.

Es zeigen in schematischer Darstellung:
Figur 1 - ein medizinisches Gerät zur Speisung eines HF-Chirurgischen Instruments sowie ein Neutralleiterkabel zur Verbindung einer Neutralleiteranordnung mit dem Gerät in schematischer Darstellung,
Figur 2 - eine Ausführungsform der erfindungsgemäßen Instrumententestanordnung,
Figuren 2a, 2b - eine Darstellung der Kopplung der Zündtestelektrode an die Neutralleiter gemäß einer erfindungsgemäßen Instrumententestanordnung,
Figuren 3a, 3b - Ansichten einer Ausführungsform der erfindungsgemäßen Instrumententestanordnung mit einer ringförmigen Zündtestelektrode,
Figuren 4a, 4b, 5 - weitere Ausführungsformen der erfindungsgemäßen Instrumententestanordnung,
Figuren 6 bis 10 - weitere Ausführungsformen der erfindungsgemäßen Instrumententestanordnung,
Figuren 11 bis 13 - spezielle Ausgestaltungen der Ausführungsformen gemäß den Figuren 6 bis 9.

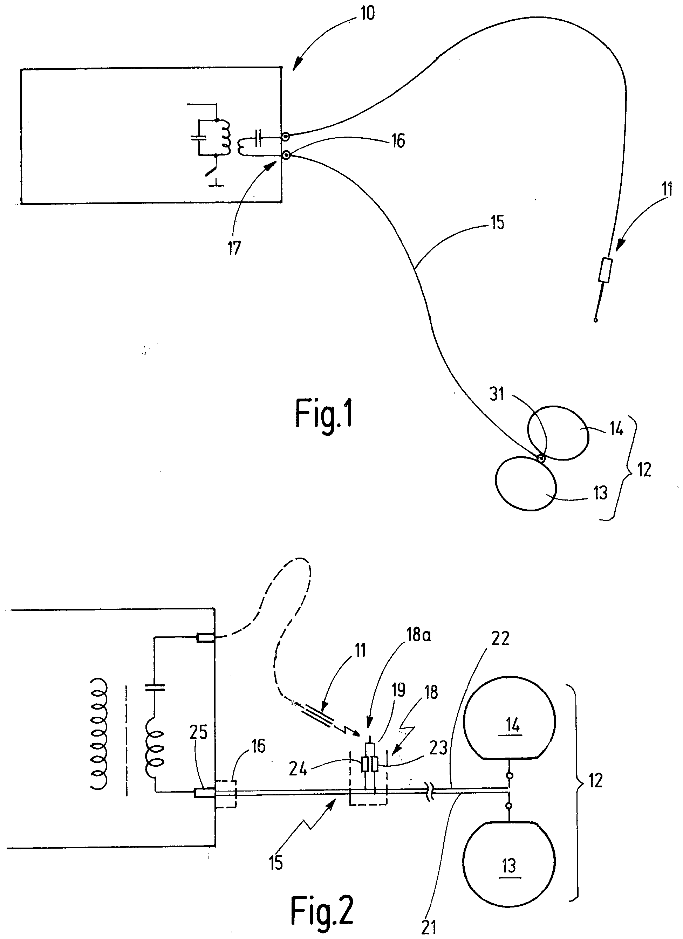

In Figur 1 ist ein medizinisches Gerät 10 zur Speisung eines elektrochirurgischen Instruments 11 veranschaulicht, das auf biologisches Gewebe einwirkt. Bei dem Instrument 11 kann es sich um ein offenchirurgisch, laparoskopisch oder auch endoskopisch zu nutzendes Instrument, z.B. eine sogenannte Sonde, handeln. Insbesondere kann das Instrument 11 eine Argon-Plasma-Koagulationssonde sein.

Der mit dem Instrument 11 in das biologische Gewebe eingeleitete Strom wird über eine Neutralelektrodenanordnung 12 zu dem Gerät 10 zurückgeführt. Die Neutralelektrodenanordnung 12 umfasst zwei oder mehrere Teilelektroden 13, 14, die zu einem Neutralleiterkabel 15 gehören oder an dieses angeschlossen sind. Das Neutralleiterkabel 15 stellt eine flexible Verbindung zwischen der Neutralelektrodenanordnung 12 und dem Gerät 10 her. An seinem geräteseitigen Ende weist das Neutralleiterkabel 15 einen Gerätestecker 16 auf, der in die Neutralbuchse 17 des Geräts 10 eingesteckt ist.

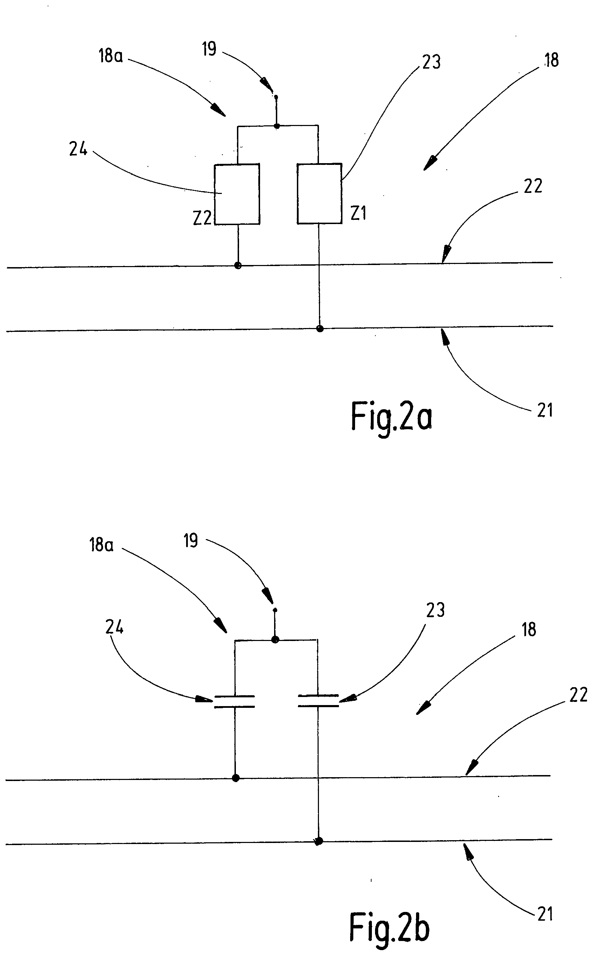

Wie Figur 2 zeigt, kann das Neutralleiterkabel 15 mit einer Instrumententestanordnung 18 versehen sein, die dazu dient, die Zünd- und Funktionsfähigkeit des Instruments 11 prüfen zu können.

Die Instrumententestanordnung 18 weist eine Zündtestelektrode 19 auf, die elektrisch sowohl mit einem ersten Neutralleiter 21 als auch mit einem zweiten Neutralleiter 22 des Neutralleiterkabels 15 verbunden ist. Der erste Neutralleiter 21 und der zweite Neutralleiter 22 sind voneinander isoliert, so dass eine Gleichstromleitung zwischen ihnen nicht stattfindet. Die Zündtestelektrode 19 ist mit dem Neutralleiter 21 über ein erstes Impedanzbauelement 23, beispielsweise ein kapazitives oder auch über ein resistives, z.B. ein ohmsches Bauelement verbunden. Die Zündtestelektrode 19 ist außerdem über ein zweites Impedanzbauelement 24 mit dem zweiten Neutralleiter 22 verbunden. Die Impedanzen der Impedanzbauelemente 23, 24 sind vorzugsweise gleich.

Der Neutralleiter 21 ist mit der ersten Teilelektrode 13 verbunden und der Neutralleiter 22 ist mit der zweiten Teilelektrode 14 verbunden. Eine Splitschaltung 25 ist in Figur 2 lediglich schematisch angedeutet und dient dazu, den ohmschen Widerstand oder die Impedanz zwischen den beiden Teilelektroden 13, 14 zu messen. Auf diese Weise wird erfasst, ob beide Teilelektroden 13, 14, und somit die gesamte Neutralelektrodenanordnung 12, einen ausreichenden elektrischen Kontakt zum Patienten aufweist.

Das Gerät 10 und das Instrument 11 sowie insbesondere die Instrumententestanordnung 18 arbeiten wie folgt:

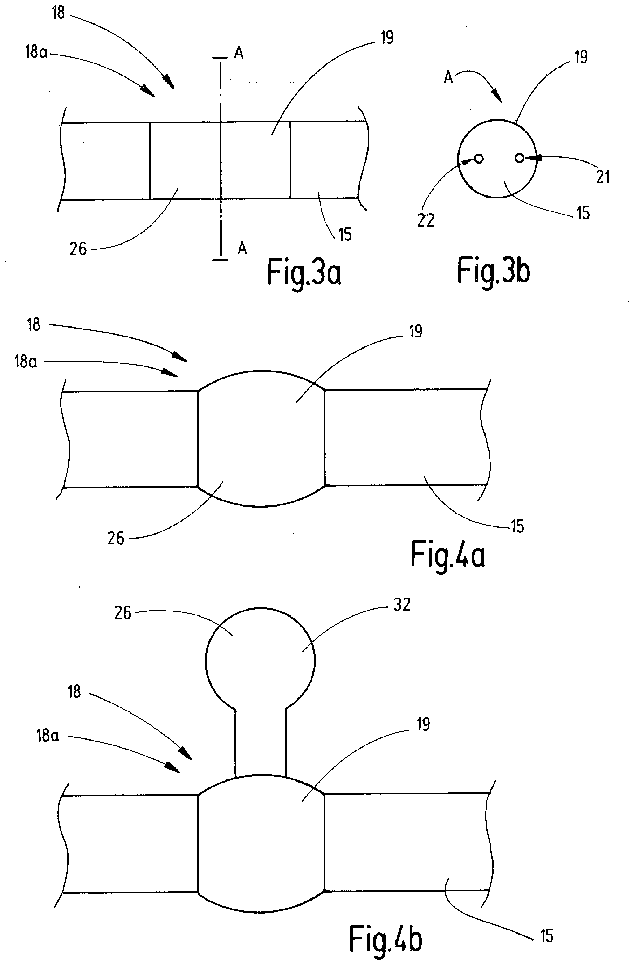

Um eine Behandlung mit dem Instrument 11 durchzuführen, wird dieses zunächst, wie in Figur 1 dargestellt, mit dem Gerät 10 verbunden. Außerdem wird die Neutralelektrodenanordnung 12 an dem menschlichen oder tierischen Patienten angebracht. Der Anwender wird dann die Funktions- und insbesondere die Zündfähigkeit des Instruments 11 überprüfen wollen. Dazu bringt er das distale Ende der Sonde oder des Instruments 11 in die Nähe der Zündtestelektrode 19 bzw. einer Testfläche 26 (Figur 3), welche die Zündtestelektrode 19 umfasst, und aktiviert dann den Generator des Geräts 10. Bei zündfähigem Instrument 11 springt ein Funke zu der Zündtestelektrode 19. Der testweise fließende Strom fließt etwa zu gleichen Teilen über beide Leiter 21, 22 zu dem Gerät 10. Die Neutralelektrodenanordnung 12 bleibt auf einheitlichem, frei floatenden Potential. Zwischen beiden Teilelektroden entsteht keine Potentialdifferenz. Wegen der gleichmäßigen Stromaufteilung erkennt die Splitschaltung 25 kein Fehlersignal. Sie sieht die gleichen Verhältnisse wie beim Eingriff oder einer Behandlung beim Patienten.

In Figur 2a ist die Instrumententestanordnung 18 aus Figur 2 verallgemeinert dargestellt. Die Zündtestelektrode 19 weist sowohl eine Verbindung zu dem ersten Neutralleiter 21 als auch eine Verbindung zu dem zweiten Neutralleiter 22 auf. Die Verbindung der Zündtestelektrode 19 zu dem ersten Neutralleiter 21 bzw. zu dem zweiten Neutralleiter 22 ist durch eine erste Impedanz 23 (Z1) bzw. eine zweite Impedanz 24 (Z2) charakterisiert. Diese Impedanzen können wie in dem Ausführungsbeispiel nach Figur 2 durch diskrete elektrische Bauelemente verwirklicht sein. Eine kapazitive Kopplung wie in der Instrumententestanordnung in Figur 2b, kann aber beispiels- und vorzugsweise auch durch die Kapazität gebildet werden, die, vermittelt durch die Kabelisolierung als Dielektrikum, zwischen einem metallischen Element an dem Neutralleiterkabel 15 und dessen Neutralleitern 21, 22 gebildet wird.

Das metallische Element kann als Ring um das Neutralleiterkabel 15 ausgebildet sein, wie im Zusammenhang mit Figur 3 dargestellt ist. Der Ring koppelt durch die Kabelisolation kapazitiv sowohl mit dem ersten Neutralleiter 21 als auch mit dem zweiten Neutralleiter 22.

Unabhängig davon, ob die kapazitive Kopplung zwischen der Zündtestelektrode 19 und den Neutralleitern 21, 22 durch diskrete Bauelemente (Kondensatoren) oder die Streukapazität zwischen der Zündtestelektrode 19 und den Neutralleitern 21, 22 gebildet ist, ist die Kapazität gering. Vorzugsweise beträgt die zusätzliche Kapazität zwischen den Neutralleitern 21, 22 höchstens 10 nF, weiter vorzugsweise lediglich 5 nF und im besten Falle höchstens 1 nF. Die Kapazität ist vorzugsweise so gering bemessen, dass die von der Kapazität gebildete Impedanz strombegrenzend wirkt, um gefährliche oder auch nur unangenehme elektrische Durchströmungen des Operationspersonals zu verhindern. Berührt eine Person den Patienten einerseits am Operationsort oder die Masse und andererseits die Zündtestelektrode 19, ist der Strom durch die Person infolge der Kleinheit der Kapazität auf ein ungefährliches Maß beschränkt. Vorzugsweise ist die Impedanz größer als 400 V/A.

Unabhängig davon wie die Kopplung der Zündtestelektrode mit den Neutralleitern ausgestaltet ist, sind die Impedanzen von der Zündtestelektrode 19 zu den Neutralleitern 21, 22 vorzugsweise gleich oder höchstens leicht unterschiedlich. Auf diese Weise führt ein Testfunken nicht zu einem Auslösen der Splitschaltung 25.

Über das in Figur 3a dargestellte Neutralleiterkabel 15 kann zur Ausbildung einer Instrumententestanordnung 18 eine ringförmige Zündtestelektrode 19 gezogen sein. Die ringförmige Zündtestelektrode 19 ist vorzugsweise ringförmig geschlossen, kann jedoch auch unterbrochen sein. Bei der Zündtestelektrode 19 kann es sich um einen Metallring oder um eine Metallfolie oder eine Metallbeschichtung, etwa eine Metallisierung oder eine Bedampfung des Neutralleiterkabels mit Metall handeln. Indem die ringförmige Zündtestelektrode 19 das Neutralleiterkabel 15 umgreift, kann die Zündtestelektrode 19 kapazitiv ausreichend stark und gleichmäßig mit beiden Neutralleitern 21, 22 koppeln.

Die Zündtestelektrode 19 weist eine Testfläche 26 auf. Der Verwender des chirurgischen Instruments 11 bringt das Instrument 11 in die Nähe der Testfläche 26, um die Zündfähigkeit und sonstige Funktionsfähigkeit des Instruments 11 zu überprüfen. Die Zündtestelektrode 19 weist in der Ausführungsform der Figur 3a eine zusammenhängende Testfläche 26 auf. Die Testfläche 26 lässt sich auf diese Weise besonders gut reinigen.

Figur 4a zeigt, ebenso wie Figur 3, ein Neutralleiterkabel 15 mit einer Zündtestelektrode 19, die ringförmig ist, in diesem Fall jedoch eine nach außen gewölbte, konvexe Testfläche 26 aufweist. Die Testfläche 26 ist ebenfalls zusammenhängend. Durch die zweidimensional, d.h. in zwei Richtungen nach außen gewölbte Form und auch durch die zusammenhängende Oberfläche lässt sich die Zündtestelektro-de 19 leicht reinigen. Der Nutzer der Instrumententestan-ordnung 18 hat zudem bei der Durchführung des Instrumententests die relative Lage der Sonde und der Zündtestelektrode 19 stets im Blick und kann, falls gewünscht, ausschließen, dass er die Zündtestelektrode 19 mit der Sonde 11 berührt und diese womöglich kontaminiert.

Eine ringförmige Zündtestelektrode 19, z.B. eine wie in den Figuren 3 und 4a dargestellte, kann einen quer zu dem Neutralleiterkabel 15 abstehenden Fortsatz 32, beispielsweise einen kugelförmigen, pilzförmigen oder türknaufförmigen Fortsatz 32, aus Metall aufweisen. Auf diese Weise ergibt sich ein gewisser Abstand zwischen dem Neutralleiterkabel 15 und der an dem Fortsatz 32 angeordneten Testfläche 26. Ein Beispiel für eine Zündtestelektrode mit einem Fortsatz 32 und einem Ring ist in Figur 4b dargestellt.

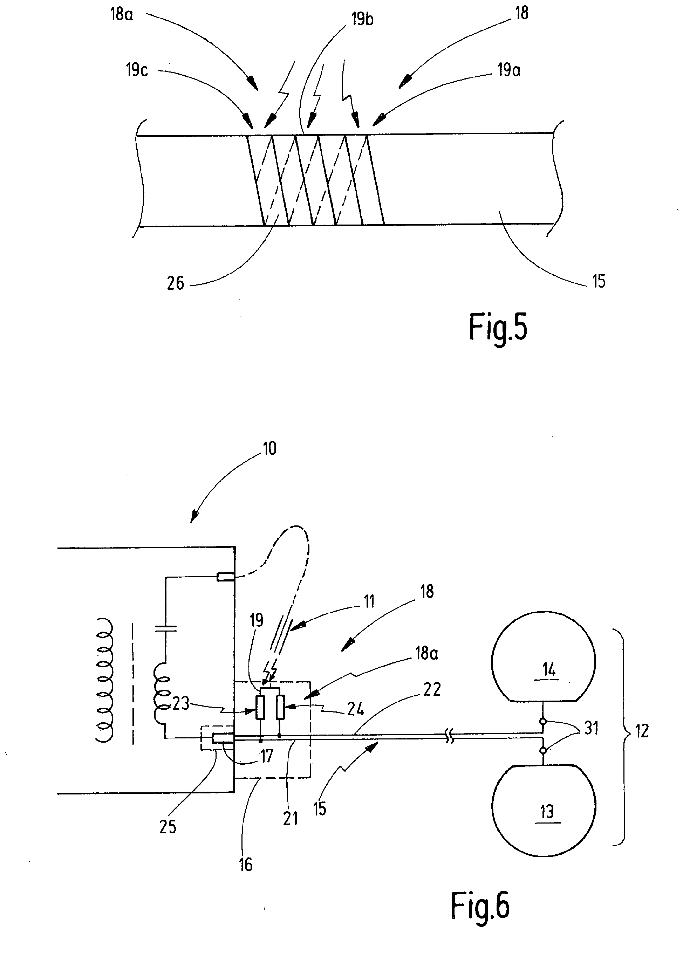

Das in Figur 5 dargestellte Neutralleiterkabel 15 ist von einer alternativen spiralförmigen, nach Art einer Schraubenfeder ausgebildeten Zündtestelektrode 19 umgeben. Die Zündtestelektrode 19 kann durch ein einer Schraubenlinie folgendes Metallband, Metallisierung oder Folie handeln. Durch die schraubenlinienförmige Ausgestaltung der Zündtestelektrode 19 kann eine besonders flexible Zündtestelektrode geschaffen werden, die die Flexibilität des Neutralleiterkabels 15 kaum beeinträchtigt. Die Kopplung der Zündtestelektrode 19 mit den Neutralleitern 21, 22 kann wiederum kapazitiv mit der Kabelisolierung als Dielektrikum oder auf jede andere in dieser Schrift beschriebenen Art erfolgen.

Die Zündtestelektrode 19 in Figur 5 stellt sich aus Sicht des Funkens, wie in der Figur angedeutet, wie zwei oder mehrere Zündtestelektroden 19 a, b, c dar, die untereinander galvanisch verbunden sind. Es kann hier auch von miteinander leitfähig verbundenen Teilen oder Abschnitten einer einzigen Zündtestelektrode gesprochen werden.

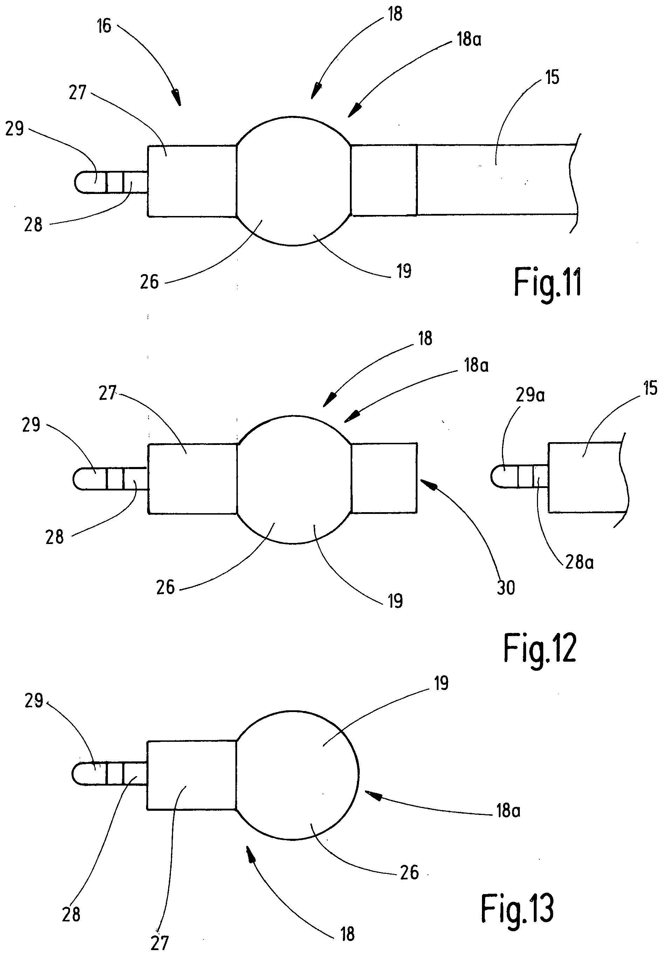

Figur 6 stellt eine Instrumententestanordnung 18 mit einer Zündtestelektrode 19 in einem Gerätestecker 16 dar, der in Figur 2 schematisch gestrichelt angedeutet ist. Eine beispielhafte Ausführungsform des Gerätesteckers ist in Figur 11 dargestellt. Der Gerätestecker 16 weist einen Körper 27 auf. Der Körper 27 wird von einem Metallring umfasst, der die Zündtestelektrode 19 bildet. Der Ring ist zusätzlich in Längsrichtung des Gerätesteckers 16 gewölbt, so dass eine zweidimensional gewölbte zusammenhängende Testfläche 26 gebildet wird.

Bevorzugt koppelt die Zündtestelektrode 19 bei den oben beschriebenen Ausführungsbeispielen kapazitiv auf die in dem Neutralleiterkabel 15 bzw. dem Stecker des Neutralleiterkabels angeordneten Neutralleitern 21, 22 gleichmäßig durch den Isolatorkörper 27. Die Kopplung bzw. Verbindung der Neutralelektrode mit den Neutralleitern 21, 22 kann aber beispielsweise auch durch kapazitive Bauelemente erfolgen, die in dem Isolatorkörper 27 angeordnet sein können. Der Stecker 16 weist Kontakte 28, 29 eines von dem Gerätestecker 16 wegragenden Anschlussstiftes auf, die mit den Neutralleitern 21, 22 kontaktiert sind. Der Anschlussstift wird bei der Verwendung in die Neutralelektroden-Buchse 17 des Geräts 10 gesteckt. Ansonsten gilt das für die anderen Ausführungsformen Beschriebene.

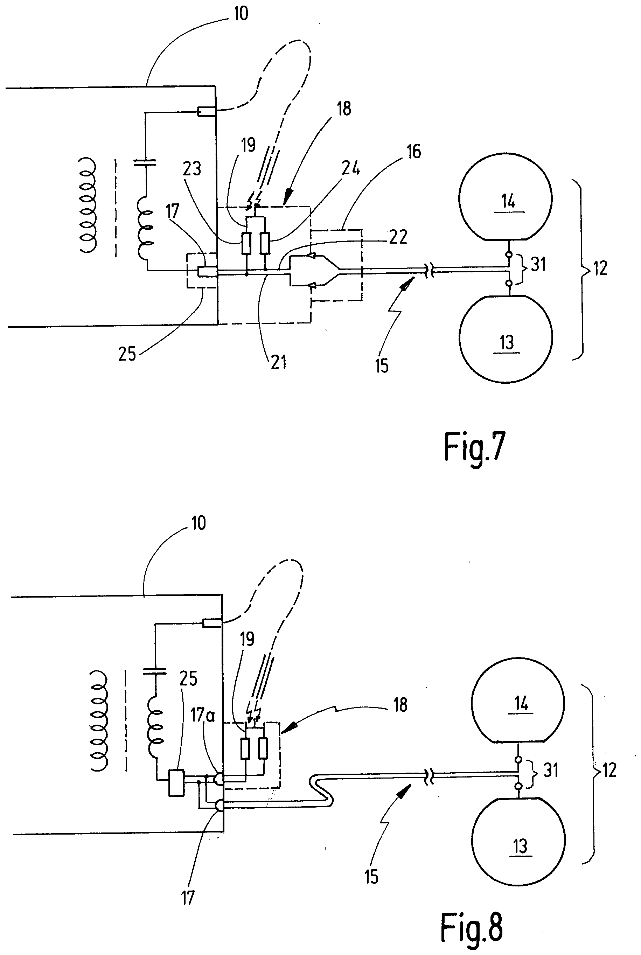

In einer weiteren Ausführungsform gemäß Figur 7 ist die Instrumententestanordnung 18 als Zwischenstecker ausgebildet, der zwischen dem Gerätestecker 16 und der Neutralbuchse 17 angeordnet werden kann. Ein Beispiel für den in Figur 7 schematisch dargestellten Zwischenstecker ist in Figur 12 gegeben. Der Zwischenstecker weist einen Isolatorkörper 27 auf, auf dem eine ringförmige Zündtestelektrode 19 angeordnet ist. Die Zündtestelektrode 19 ist ähnlich wie die entsprechende Elektrode in dem Gegenstand aus Figur 11 ausgebildet. Insbesondere weist sie eine konvexe Wölbung in Längsrichtung des Steckers auf, so dass eine insgesamt zweidimensionale Wölbung der Testfläche 26 erreicht wird. Die Zündtestelektrode 19 koppelt beispielsweise kapazitiv über Kondensatoren oder über den Isolatorkör-per 27 gleichmäßig auf Verlängerungen der Neutralleiter 21, 22, die in dem Zwischenstecker angeordnet sind und mit den Kontakten 28, 29 verbunden sind. Der Zwischenstecker weist auf der dem Anschlussstift gegenüberliegenden Seite eine Buchse 30 auf, die Kontakte enthält, die mit den in dem Zwischenstecker verlaufenden Neutralleitern kontaktiert ist. Das Neutralkabel 15 weist an einem Ende eine Anschlusseinrichtung mit einem Anschlussstift mit Kontakten 28a, 29a auf. Der Anschlussstift wird in die Buchse 30 gesteckt, so dass die Teilelektroden 13, 14 an das Gerät 10 angeschlossen werden. Ansonsten gilt das für die anderen Ausführungsformen Beschriebene.

In Figur 8 ist eine weitere Ausführungsform der erfindungsgemäßen Instrumententestanordnung 18 dargestellt. An dem Gerät ist eine gesonderte Buchse 17a ausgebildet, die elektrisch parallel zu der Buchse 17 geschaltet ist. In diese gesonderte Buchse 17a kann wiederum die Instrumententestanordnung 18 eingesteckt werden. Ein Beispiel für einen solchen Zündteststecker zeigt Figur 13. Die Instrumententestanordnung 18 weist wiederum einen Anschlussstift mit Kontakten 28, 29 auf, die beispielsweise jeweils kapazitiv mit der knaufförmigen Elektrode 19 koppeln, die an einem dem Anschlussstift gegenüberliegenden Ende des Isolatorkör-pers 27 angeordnet ist. Von den Besonderheiten dieser Ausführungsform abgesehen, gilt das bereits im Zusammenhang mit anderen Ausführungsformen Beschriebene.

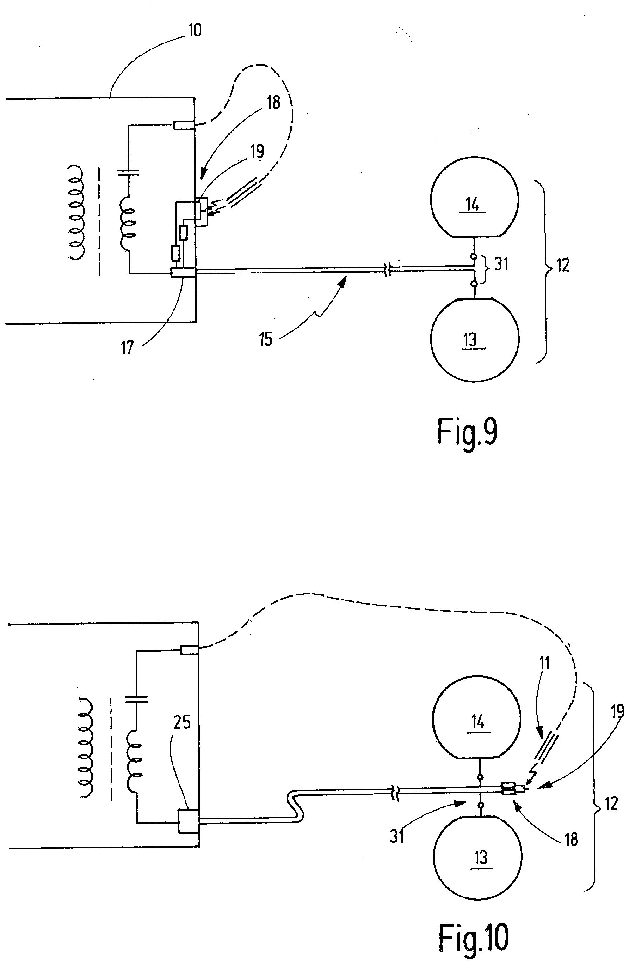

Es ist auch möglich, die Instrumententestanordnung 18 wie in Figur 9 dargestellt in das Gerät 10 einzubauen. Die Zündtestelektrode 19 kann beispielsweise ebenso wie in dem Gegenstand der Figur 13 knaufförmig oder kugelförmig gewölbt sein. Ergänzend wird auf die vorherige Beschreibung verwiesen.

Es ist auch möglich, die Instrumententestanordnung 18 an der Neutralelektrodenanordnung 12, beispielsweise an dem Neutralelektrodenanschluss 31 anzuordnen. Die Instrumententestanordnung 18 kann ein eigenes Isolatorgehäuse aufweisen oder z.B. in dem Neutralelektrodenanschluss 31 angeordnet sein. Wiederum gilt, von den Besonderheiten dieser Ausführungsform abgesehen, das bereits im Zusammenhang mit anderen Ausführungsformen Beschriebene.

Es wird eine Instrumententestanordnung 18 angegeben, die vorzugsweise für medizinische Instrumente 11 zur Argon-Plasma-Koagulation eingerichtet ist. Die Instrumententestanordnung 18 weist eine Elektrodenanordnung 18a, mit mindestens einer Zündtestelektrode 19 auf. Eine Zündtestelektrode ist mit zwei Neutralleitern 21, 22 eines Neutralleiterkabels 15 oder Geräts 10 verbunden, wobei die Neutralleiter 21, 22 gegeneinander isoliert sind, und wobei die erste Kopplungsimpedanz 23 zwischen der Zündtestelektrode 19 und dem ersten Neutralleiter 21 und die zweite Kopplungsimpedanz 24 zwischen der Zündtestelektrode 19 und dem zweiten Neutralleiter 22 gleich oder höchstens leicht unterschiedlich sind, so dass eine einer Neutralelektrodenanordnung 12 zugeordnete Splitschaltung 25 auf Grund eines Testzündfunkens nicht auslöst.

### Bezugszeichenliste:

- 10: Medizinisches Gerät
- 11: Elektrochirurgisches Instrument
- 12: Neutralelektrodenanordnung
- 13, 14: Teilelektroden
- 15: Neutralleiterkabel
- 16: Gerätestecker
- 17: Neutralbuchse
- 18: Instrumententestanordnung
- 18a: Elektrodenanordnung
- 19, 19: Zündtestelektrode
- a, b, c:
- 21: Erster Neutralleiter
- 22: Zweiter Neutralleiter
- 23, Z1: Erstes Impedanzbauelement/ erste Impedanz
- 24, Z2: Zweites Impedanzbauelement/ zweite Impedanz
- 25: Splitschaltung
- 26: Testfläche
- 27: Isolatorkörper
- 28, 29, a: Kontakte
- 30: Buchse
- 31: Neutralelektrodenanschluss
- 32: Fortsatz

## Patentansprüche

1. Instrumententestanordnung (18) insbesondere für medizinische Instrumente (11) zur Argon-Plasma-Koagulation,
mit einer Elektrodenanordnung (18a), die mindestens eine Zündtestelektrode (19) aufweist, die elektrisch mit einem Neutralleiter (21, 22) eines Neutralleiterkabels (15) oder eines Geräts (10) verbunden ist,
wobei das flexible Neutralleiterkabel (15) oder das Gerät (10) zwei Neutralleiter (21, 22) aufweist, die gegeneinander isoliert und mit zwei Teilelektroden (13, 14) einer Neutralelektrodenanordnung (12) verbunden sind, wobei die Teilelektroden (13, 14) an eine Splitschaltung (25) angeschlossen sind, die dazu dient, den elektrischen Widerstand zwischen den beiden Teilelektroden (13, 14) zu messen, um zu erfassen, ob die Teilelektroden (13, 14) einen ausreichenden elektrischen Kontakt zu dem Patienten aufweisen,
wobei die Zündtestelektrode (19) zu den Neutralleitern (21, 22) mit gleichen oder höchstens leicht unterschiedlichen Kopplungsimpedanzen (23, 24) koppelt, so dass bei Beaufschlagung der Zündtestelektrode (19) mit HF-Energie keine so unterschiedlichen Ströme in den Neutralleitern (21, 22) fließen, dass die Splitschaltung (25) bei Beaufschlagung der Zündtestelektrode (19) mit HF-Energie fälschlicherweise einen mangelnden Kontakt der Teilelektroden (13, 14) zu dem Körper des Patienten anzeigt.

2. Instrumententestanordnung (18) nach Anspruch 1, wobei eine Zündtestelektrode (19) mit wenigstens zwei Neutralleitern (21, 22) verbunden ist.

3. Instrumententestanordnung (18) nach einem der vorherigen Ansprüche, wobei die Zündtestelektrode (19) das Neutralleiterkabel (15) wenigstens teilweise umgreift.

4. Instrumententestanordnung (18) nach einem der vorherigen Ansprüche, wobei die Zündtestelektrode (19) ein metallischer Ring oder eine Hülse ist.

5. Instrumententestanordnung (18) nach einem der vorherigen Ansprüche, wobei die Zündtestelektrode (19) eine Metallfolie, ein Draht eine Metallisierung und/oder eine Metallbeschichtung ist.

6. Instrumententestanordnung (18) nach Anspruch 5,
**dadurch gekennzeichnet, dass** die Zündtestelektrode (19) als Schraubenwendel ausgebildet ist, deren Windungen die Isolierung des Neutralleiterkabels umgreifen.

7. Instrumententestanordnung nach einem der vorstehenden Ansprüche, **dadurch gekennzeichnet, dass** die Zündtestelektrode (19) über ein eine Impedanz aufweisendes Bauelement (23, 24) mit dem Neutralleiter (21, 22) verbunden ist.

8. Instrumententestanordnung nach Anspruch 7, **dadurch gekennzeichnet, dass** das Bauelement (23, 24) eine Kapazität aufweist.

9. Instrumententestanordnung nach Anspruch 7, **dadurch gekennzeichnet, dass** das Bauelement (23, 24) ein ohmscher Widerstand ist.

10. Instrumententestanordnung nach einem der vorherigen Ansprüche, **dadurch gekennzeichnet**, die Zündtestelektrode (19) über ein eine Impedanz aufweisendes erstes Bauelement (23) mit einem ersten Neutralleiter (21) und über ein eine Impedanz aufweisendes zweites Bauelement (24) mit einem zweiten Neutralleiter (22) verbunden ist, wobei das erste und das zweite Bauelement (23, 24) gleiche elektrische Kennwerte aufweisen.

11. Instrumententestanordnung (18) nach einem der vorherigen Ansprüche, **dadurch gekennzeichnet, dass** die Gesamtkapazität zwischen den Neutralleitern in der Instrumententestanordnung (18) kleiner oder gleich 1 nF ist.

12. Instrumententestanordnung nach einem der vorherigen Ansprüche, **dadurch gekennzeichnet, dass** die Elektrodenanordnung (18a) an einem flexiblen Neutralleiterkabel (15) zwischen einem Gerätestecker (16) und einer Neutralelektrodenanordnung (12) oder einem Neutralelektrodenanschluss (31) angeordnet ist.

13. Instrumententestanordnung nach einem der vorherigen Ansprüche, **dadurch gekennzeichnet, dass** die Zündtestelektrode eine konvexe Testfläche (26) aufweist.

14. Instrumententestanordnung nach einem der vorherigen Ansprüche, **dadurch gekennzeichnet, dass** die Zündtestelektrode (19) eine zusammenhängende Testfläche (26) aufweist.

## Claims

1. Instrument test arrangement (18), in particular for medical instruments (11) for argon plasma coagulation,
with an electrode arrangement (18a) which has at least one ignition test electrode (19) that is electrically connected to a neutral conductor (21, 22) of a neutral conductor cable (15) or of a device (10),
wherein the flexible neutral conductor cable (15) or the device (10) has two neutral conductors (21, 22) which are isolated from each other and connected to two partial electrodes (13, 14) of a neutral electrode arrangement (12), wherein the partial electrodes (13, 14) are connected to a split circuit (25) which serves to measure the electrical resistance between the two partial electrodes (13, 14) in order to detect whether the partial electrodes (13, 14) have an adequate electrical contact with the patient,
wherein the ignition test electrode (19) is coupled to the neutral conductors (21, 22) with the same or at most slightly different coupling impedances (23, 24) so that when the ignition test electrode (19) is loaded with HF energy, the currents flowing in the neutral conductors (21, 22) are not so different that the split circuit (25, when the ignition test electrode (19) is loaded with HF energy, falsely indicates a lack of contact of the partial electrodes (13, 14) with the body of the patient.

2. Instrument test arrangement (18) according to claim 1, wherein an ignition test electrode (19) is connected to at least two neutral conductors (21, 22).

3. Instrument test arrangement (18) according to one of the preceding claims, wherein the ignition test electrode (19) at least partially surrounds the neutral conductor cable (15).

4. Instrument test arrangement (18) according to any of the preceding claims, wherein the ignition test electrode (19) is a metallic ring or a sleeve.

5. Instrument test arrangement (18) according to any of the preceding claims, wherein the ignition test electrode (19) is a metal film, a wire, a metallisation, and/or a metal coating.

6. Instrument test arrangement (18) according to claim 5, **characterised in that** the ignition test electrode (19) is configured as a helical coil, the windings of which surround the insulation of the neutral conductor cable.

7. Instrument test arrangement according to any of the preceding claims, **characterised in that** the ignition test electrode (19) is connected to the neutral conductor (21, 22) via a component (23, 24) having an impedance.

8. Instrument test arrangement according to claim 7, **characterised in that** the component (23, 24) has a capacitance.

9. Instrument test arrangement according to claim 7, **characterised in that** the component (23, 24) is an ohmic resistor.

10. Instrument test arrangement according to any of the preceding claims, **characterised in that** the ignition test electrode (19) is connected to a first neutral conductor (21) via a first component (23) having an impedance, and to a second neutral conductor (22) via a second component (24) having an impedance, wherein the first and the second components (23, 24) have the same electrical characteristic values.

11. Instrument test arrangement (18) according to any of the preceding claims, **characterised in that** the total capacitance between the neutral conductors in the instrument test arrangement (18) is less than or equal to 1 nF.

12. Instrument test arrangement according to any of the preceding claims, **characterised in that** the electrode arrangement (18a) is arranged on a flexible neutral conductor cable (15) between a device plug (16) and a neutral electrode arrangement (12) or a neutral electrode connection (31).

13. Instrument test arrangement according to any of the preceding claims, **characterised in that** the ignition test electrode has a convex test surface (26).

14. Instrument test arrangement according to any of the preceding claims, **characterised in that** the ignition test electrode (19) has a cohesive test surface (26).

## Revendications

1. Dispositif de test d'instruments (18) destiné notamment à des instruments médicaux (11) pour la coagulation au plasma d'argon,
comprenant un agencement d'électrode (18a) qui présente au moins une électrode de test d'allumage (19), laquelle est reliée électriquement à un conducteur neutre (21, 22) d'un câble à conducteurs neutres (15) ou d'un appareil (10),
le câble à conducteurs neutres (15) souple ou l'appareil (10) présentant deux conducteurs neutres (21, 22) qui sont isolés l'un vis-à-vis de l'autre et sont reliés à deux électrodes élémentaires (13, 14) d'un agencement d'électrode neutre (12), les électrodes élémentaires (13, 14) étant raccordées à un circuit diviseur (25) qui sert à mesurer la résistance électrique entre les deux électrodes élémentaires (13, 14) pour détecter si les électrodes élémentaires (13, 14) présentent un contact électrique suffisant avec le patient,
l'électrode de test d'allumage (19) étant couplée avec les conducteurs neutres (21, 22) qui ont des impédances de couplage (23, 24) identiques ou tout au plus légèrement différentes, de sorte que lors de l'application d'une énergie HF à l'électrode de test d'allumage (19), les courants circulant dans les conducteurs neutres (21, 22) ne sont pas si différents que, lors de l'application d'énergie HF à l'électrode de test d'allumage (19), le circuit diviseur (25) indique de façon erronée un contact insuffisant des électrodes élémentaires (13, 14) avec le corps du patient.

2. Dispositif de test d'instruments (18) selon la revendication 1, dans lequel une électrode de test d'allumage (19) est reliée à au moins deux conducteurs neutres (21, 22).

3. Dispositif de test d'instruments (18) selon l'une des revendications précédentes, dans lequel l'électrode de test d'allumage (19) entoure au moins en partie le câble à conducteurs neutres (15).

4. Dispositif de test d'instruments (18) selon l'une des revendications précédentes, dans lequel l'électrode de test d'allumage (19) est une bague métallique ou un manchon.

5. Dispositif de test d'instruments (18) selon l'une des revendications précédentes, dans lequel l'électrode de test d'allumage (19) est une feuille métallique, un fil métallique, une métallisation et/ou un revêtement métallique.

6. Dispositif de test d'instruments (18) selon la revendication 5, **caractérisé en ce que** l'électrode de test d'allumage (19) est réalisée sous la forme d'une hélice dont les spires entourent l'isolation du câble à conducteurs neutres.

7. Dispositif de test d'instruments selon l'une des revendications précédentes, **caractérisé en ce que** l'électrode de test d'allumage (19) est reliée au conducteur neutre (21, 22) par l'intermédiaire d'un composant (23, 24) présentant une impédance.

8. Dispositif de test d'instruments selon la revendication 7, **caractérisé en ce que** le composant (23, 24) présente une capacité.

9. Dispositif de test d'instruments selon la revendication 7, **caractérisé en ce que** le composant (23, 24) est une résistance ohmique.

10. Dispositif de test d'instruments selon l'une des revendications précédentes, **caractérisé en ce que** l'électrode de test d'allumage (19) est reliée à un premier conducteur neutre (21), par l'intermédiaire d'un premier composant (23) présentant une impédance, et à un deuxième conducteur neutre (22), par l'intermédiaire d'un deuxième composant (24) présentant une impédance, le premier et le deuxième composant (23, 24) présentant des caractéristiques électriques identiques.

11. Dispositif de test d'instruments (18) selon l'une des revendications précédentes, **caractérisé en ce que** la capacité totale entre les conducteurs neutres dans le dispositif de test d'instruments (18) est inférieure ou égale à 1 nF.

12. Dispositif de test d'instruments selon l'une des revendications précédentes, **caractérisé en ce que** l'agencement d'électrode (18a) est disposé sur un câble à conducteurs neutres (15) souple, entre un connecteur d'appareil (16) et un agencement d'électrodes neutres (12) ou une borne d'électrode neutre (31).

13. Dispositif de test d'instruments selon l'une des revendications précédentes, **caractérisé en ce que** l'électrode de test d'allumage présente une surface de test (26) convexe.

14. Dispositif de test d'instruments selon l'une des revendications précédentes, **caractérisé en ce que** l'électrode de test d'allumage (19) présente une surface de test (26) continue.
